# EUROPEAN PATENT SPECIFICATION

(11) **EP 1 712 234 B1**
(45) Date of publication and mention of the grant of the patent: **28.01.2009**
(21) Application number: 06114903.5
(22) Date of filing: 11.09.2002
(51) Int. Cl.: A61K 31/50, A61P 35/00

(54) **Use of 4-Pyridylmethylphthalazines for Renal Cancer Treatment**
Die Verwendung von 4-Pyridylmethylphthalazinen zur Behandlung von Nierentumoren
L'utilisation de 4-pyridylmethylphthalazines pour le traitement de cancer de reins

(30) Priority: 12.09.2001 US 331025 P; 12.09.2001 US 318694 P; 14.09.2001 US 322044 P; 12.06.2002 US 388163 P
(43) Date of publication of application: 18.10.2006
(62) Divisional of application: 02779338.9
(73) Proprietor: NOVARTIS AG, 4056 Basel (CH); Novartis Pharma GmbH, 1230 Vienna (AT)
(72) Inventor: DUGAN, Margaret Han, NY (US)
(74) Representative: Graff, Alan

(56) References cited:
- EP-A- 0 722 936
- WO-A-01/10859
- WO-A-98/35958
- WO-A-02/090346

## Description

The present invention relates to the use of 4-pyridylmethyl-phthalazine agents in the manufacture of a medicament for the treatment of a renal cancer.

4-Pyridylmethyl-phthalazine derivatives that are selective inhibitors of VEGF receptor tyrosine kinase and their preparation, pharmaceutical formulations thereof and methods of making such compounds are described in WO00/59509, EP02/04892, WO01/10859 and, in particular, in U.S. Patent No. 6,258,812. Such compounds reduce the microvasculature and inhibit growth of primary tumors and metastases in animal models and are useful for treating diseases associated with deregulated angiogenesis, especially neoplastic diseases (solid tumors), such as breast cancer, cancer of the colon, lung cancer, especially small cell lung cancer, and cancer of the prostate.

Surprisingly, it was found that 4-pyridylmethyl-phthalazine agents are useful for the treatment of renal carcinoma, especially for inhibiting the metastatic growth of a renal carcinoma. Hence, the present invention relates to the use of said agents in the manufacture of a medicament for treating renal carcinoma in a patient, which comprises administering a pharmaceutically effective amount of a 4-pyridylmethyl-phthalazine agent to the patient. In particular, the present invention pertains to inhibiting metastatic growth in a patient with a renal carcinoma, which comprises administering a pharmaceutically effective amount of a 4-pyridylmethyl-phthalazine agent to the patient.

Throughout the present invention, the 4-pyridylmethyl-phthalazine agent is in particular 1-(4-chloroanilino)-4-(4-pyridylmethyl)phthalazine or a pharmaceutically acceptable salt thereof. Studies in humans have shown 1-(4-chloroanilino)-4-(4-pyridylmethyl)phthalazine to be well tolerated and to reduce tumor vascular permeability. It is understood that further references to 1-(4-chloroanilino)-4-(4-pyridylmethyl)phthalazine are intended to include pharmaceutically acceptable salts thereof.

The present invention pertains also to the use of a 4-pyridylmethyl-phthalazine agent for the manufacture of a pharmaceutical preparation for the treatment of renal carcinoma, in particular metastatic renal carcinoma, and of a pharmaceutical preparation for the inhibition of metastatic growth in a patient with a renal carcinoma.

Chemotherapy for the treatment of proliferative diseases is known in the art.

The term "metastatic growth" as used herein comprises the metastatic spread of tumors and the growth and development of micrometastases in other organs of the cancer patients.

For the determination of the active dosage of a 4-pyridylmethyl-phthalazine antiangiogenic agent to be applied to patients and, in particular, to be applied to an individual patient, two biomarkers, DCE-MRI and plasma VEGF level, along with exposure, safety, and tumor response data can be employed. For this purpose, patients, e.g., receive a continuous daily dose of, e.g., 50, 150, 300, 500, 750, 1000, 1500 or 2000 mg of 1-(4-chloroanilino)-4-(4-pyridylmethyl)phthalazine. Pharmacokinetic (PK) samples are taken at predose, and days 1, 15, and 28. DCE-MRI, which reflects the change in tumor perfusion and vascularity, are performed at baseline, day 2, and day 28. For all evaluable MRI scans, contrast enhancement for the whole tumour can be assessed by calculating the bi-directional transfer constant (Ki) and expressed as a percentage of baseline Ki for evaluation on day 2 and 28. Plasma VEGF, a proangiogenic factor produced by the tumor cells, reflects the hypoxic status of the tumor and are sampled at baseline, and on days 1, 8, 15, 22, and 28. A significant relationship exists between the percentage of baseline Ki and an increase in the dose of the 4-pyridylmethyl-phthalazine antiangiogenic agent, its exposure, and its plasma concentration as determined by the Spearman Rank Correlation. Furthermore, a significant relationship exists between the percentage reduction in Ki and the change in liver disease size at the end of cycle 2 as measured by the change in the sum of all measurable liver lesions (bi-dimensional product). Patients with non-progressive disease have a significantly greater reduction in mean ki. A 50 - 60% reduction in Ki is associated with non-progressive disease.

In general, for the treatment of renal carcinoma, the 4-pyridylmethyl-phthalazine agent can be given on a continuous basis, for example daily. For 1-(4-chloroanilino)-4-(4-pyridylmethyl)phthalazine, a daily oral dose in the range from 300 mg to 4000 mg, for example in the range from 300 mg/day to 2000 mg/day or 300 mg/day to 1000 mg/day, in particular 300, 500, 750, 1000, 1500 or 2000 mg/day, are contemplated as a pharmaceutically effective dose. However, other administration schedules are also likely to be effective and are included within the scope of the present invention. When 1-(4-chloroanilino)-4-(4-pyridylmethyl)phthalazine is administered as a pharmaceutically acceptable salt, an equivalent amount of the free base (i.e. one equivalent to the amounts described above) is administered.

A further aspect of the present invention relates to the use of improved regimens for the administration of 1-(4-chloroanilino)-4-(4-pyridylmethyl)phthalazine, or a pharmaceutically acceptable salt thereof, for the treatment of patients suffering from solid tumor diseases, including, e.g., renal cancer. According to one inventive regimen 1-(4-chloroanilino)-4-(4-pyridylmethyl)phthalazine, or a pharmaceutically acceptable salt thereof, is administered twice or more daily, for example two or three times daily, in reduced amounts compared with the known once daily administration regimens. Alternatively, the present invention embraces a treatment regimen wherein 1-(4-chloroanilino)-4-(4-pyridylmethyl)phthalazine is administered once daily at a dose in the range from 1000 mg/day to 1400 mg/day, particularly a dose of 1200 mg/day to 1300 mg/day, especially 1250 mg/day. The inventive dosing regimens reduce the toxic effects of 1-(4-chloroanilino)-4-(4-pyridylmethyl)phthalazine and improve the efficacy of the treatment by permitting an effective level of the drug, for example above about 1 micromolar, to be maintained for a longer period.

Thus, the present invention relates to administering 1-(4-chloroanilino)-4-(4-pyridylmethyl)phthalazine to a patient, which comprises administering a pharmaceutically effective amount of 1-(4-chloroanilino)-4-(4-pyridylmethyl)phthalazine, or a pharmaceutically acceptable salt thereof, to the patient on a twice daily schedule.

Alternatively, the present invention relates to administering 1-(4-chloroanilino)-4-(4-pyridylmethyl)phthalazine to a patient, which comprises administering 1-(4-chloroanilino)-4-(4-pyridylmethyl)phthalazine, or a pharmaceutically acceptable salt thereof, to the patient on a once daily schedule at a dose in the range from 1000 mg/day to 1400 mg/day, particularly a dose of 1200 mg/day to 1300 mg/day, such as 1250 mg/day.

The invention further relates to the use of the above mentioned agents for the manufacture of a medicament for treating a solid tumor disease in a patient, which comprises administering a pharmaceutically effective amount of 1-(4-chloroanilino)-4-(4-pyridylmethyl)phthalazine, or a pharmaceutically acceptable salt thereof, to the patient on a twice daily schedule, or alternatively on a once daily schedule.

1-(4-Chloroanilino)-4-(4-pyridylmethyl)phthalazine is especially useful for inhibiting metastatic growth of a cancer. Thus, the present invention further relates to inhibiting metastatic growth in a patient with a cancer, particularly a solid tumor cancer, which comprises administering a pharmaceutically effective amount of 1-(4-chloroanilino)-4-(4-pyridylmethyl)phthalazine or a pharmaceutically acceptable salt thereof, to the patient on a twice daily schedule. Alternatively, the present invention further relates to inhibiting metastatic growth in a patient with a cancer, particularly a solid tumor cancer, which comprises administering a pharmaceutically effective amount of 1-(4-chloroanilino)-4-(4-pyridylmethyl)phthalazine or a pharmaceutically acceptable salt thereof, to the patient on a once daily schedule at a dose in the range from 1000 mg/day to 1400 mg/day, particularly a dose of 1200 mg/day to 1300 mg/day, such as 1250 mg/day.

According to one aspect of the present invention, 1-(4-chloroanilino)-4-(4-pyridylmethyl)-phthalazine is given twice daily on a continuous basis, alone, or during and subsequent to other therapies, for example chemotherapy. A daily oral administration of an amount in the range from 300 mg to 4000 mg, for example in the range from 300 mg/day to 2000 mg/day or 300 mg/day to 1000 mg/day, in particular 300, 500, 750, 1000, 1500 or 2000 mg/day, split into two doses, is contemplated as a pharmaceutically effective amount in the twice daily regimen. A 1000 mg/day dose is given as two 500 mg doses 6 to 12 hours apart, for example about 8 hours apart, and a 2000 mg/day dose is administered as two 1000 mg doses 6 to 8 hours apart, for example about 12 hours apart.

In the alternative once daily dosage regimen, a dose in the range from 1000 mg/day to 1400 mg/day, particularly a dose of 1200 mg/day to 1300 mg/day, such as 1250 mg/day is administered about once per 24 hour period.

When 1-(4-chloroanilino)-4-(4-pyridylmethyl)phthalazine is administered as a pharmaceutically acceptable salt, an equivalent amount of the free base (i.e. one equivalent to the amounts described above) is administered. In this application, reference to 1-(4-chloroanilino)-4-(4-pyridylmethyl)phthalazine is intended to include pharmaceutically acceptable salts thereof.

The different aspects of the present invention can be combined freely with each other. For example, a renal cancer patient or a patient having another tumor type can be teated by administering 1-(4-chloroanilino)-4-(4-pyridylmethyl)phthalazine on a daily basis or twice daily. The improved regimens of administering 1-(4-chloroanilino)-4-(4-pyridylmethyl)-phthalazine can be applied in monotheraoy or in combination therapy, with other words, 1-(4-chloroanilino)-4-(4-pyridylmethyl)phthalazine is administered alone, or in combination with other therapeutic agents. As a combination therapy, it is administered on a once or twice daily basis as described herein and any other therapeutic agent or agents are administered according to its established administration regimen.

### Example 1

15 Patients with histologically confirmed metastatic colorectal cancer seven of which had previously received adjuvant chemotherapy are treated with a combination of 1-(4-chloroanilino)-4-(4-pyridylmethyl)phthalazine and a chemotherapy regimen. The chemotherapy regimen is 85 mg/m² of oxaplatin on day 1, 200 mg/m² of folinic acid given as a 2 hour infusion on days 1 and 2, and 5-fluorouracil administered as a bolus at a dose of 400 mg/m² followed by 600 mg/m² over 22 hours on days 1 and 2. The chemotherapy regimen is given every 14 days. 1-(4-Chloroanilino)-4-(4-pyridylmethyl)phthalazine is administered continuously as a single daily dose of 500 mg/day, 1000 mg/day or 2000 mg/day. The treatment is well-tolerated. The pharmacokinetics of oxaplatin are not altered by coadministration of 1-(4-chloroanilino)-4-(4-pyridylmethyl)phthalazine. Stable diseases are observed in 2 patients, minor responses in 3 patients and partial responses in 8 patients.

### Example 2

Ten patients having metastatic renal cell carcinoma, six of which had previous biologic therapy, are treated with 300, 750 or 1000 mg/day of 1-(4-chloroanilino)-4-(4-pyridylmethyl)phthalazine administered once a day orally. Seven of the ten patients maintain stable disease for a median of 3 months.

### Example 3

Patients with histologically confirmed advanced solid tumors and measurable disease are orally administered 1-(4-chloroanilino)-4-(4-pyridylmethyl)phthalazine twice daily until disease progression or unacceptable toxicity. Sequential cohorts of patients (3 evaluable at each dose) are treated at total daily doses of 300 mg, 500 mg, 1000 mg and 2000 mg, which was split into two separate doses administered 8 hours apart. The patients are monitored by dynamic contrast enhanced magnetic resonance imaging (DCE-MRI) of tumors to provide a measure of the bi-dimensional transfer constant (Ki) prior to treatment, on day 2 and after every 28 day cycle. In addition, tumor volume is measured by magnetic resonance imaging every 28 days, and full pharmacokinetic profiles are obtained on days 1 and 28. Treatment is well tolerated with no drug-related SAEs. The following toxicities are found among the patients administered the 300 mg, 500 mg and 1000 mg daily doses that are entered: transient grade 3 elevations of liver transaminases (2 patients), grade 1 lethargy (one patient), grade 2 lightheadedness (4 patients). DCE-MRI reveals the following reductions compared with baseline (median % reduction days 2.28 respectively 300 mg - 68.3, 500 mg - 72.98). The pharmacokinetic study shows a mean Cmin (ng/ml) and area under the curve (AUC)(h.ng.ml) respectively of 300 mg - 7.7, 82; 500 mg - 4.3, 46; 1000 mg - 27, 370. The data from the study indicates a biological effect in reducing tumor perfusion/vascular permeability and slowing tumor growth.

### Example 4

Patients with histologically confirmed advanced solid tumors and measurable disease are orally administered 1-(4-chloroanilino)-4-(4-pyridylmethyl)phthalazine once daily until disease progression or unacceptable toxicity. Sequential cohorts of patients (3 evaluable at each dose) are treated at total daily doses of 50 mg, 150mg , 300 mg, 500 mg, 750 mg, 1000 mg, 1200 mg, 1500 mg and 2000 mg administered as a single daily dose. Full Pharmacokinetic (PK) sampling is performed at predose, and days 1, 15 and 28. DCE-MRI, which reflects the change in tumor perfusion and vascular permeability, are performed at baseline, and days 2 and 28. For all evaluable MRI scans, contrast enhancement for the whole tumour are assessed by calculating the bi-directional transfer constant (Ki). Plasma VEGF, a proangiogenic factor produced by the tumor cells, reflects the hypoxic status of the tumor and is determined at baseline, and on days 1, 8, 15, 22 and 28. Of a total of 76 patients from two Phase I studies, 22 patients with colorectal cancer and liver metastasis were evaluable for DCE-MRI analysis and 63 patients with advanced cancers for plasma VEGF and PK analysis. Using SWOG response criteria, non-progressive disease was defined as ≥ 2 months stable disease.

1-(4-chloroanilino)-4-(4-pyridylmethyl)phthalazine is rapidly absorbed (Tₘₐₓ 1 to 2.5 hours).
At steady-state, which was achieved by day 15, the systemic exposure (AUC) is approximately 30% decreased compared to day 1. Dose proportional increase in exposure is observed up to 1000 mg/day. The terminal half-life is 3 to 6 hours. No dose-limiting toxicity is observed up to 2000 mg/day.

A relationship exists between the percentage of baseline Ki at days 2 and 28 and the increase in dose (day 2: p=0.01; day 28: p=0.0003), exposure (AUC; day 2: p<0.0001; day 28: p=0.003), and plasma concentration (C_{min;} day 2: p=0.0003; day 28: p<0.0001) as determined by the Spearman Rank Correlation. Furthermore, a relationship exists between the percent reduction in Ki at days 2 and 28 and the change in size of liver metastases (day 2: p=0.004; day 28: p=0.0001) at the end of day 56 as measured by the change in the sum of all measurable liver lesions (bi-dimensional product). Patients with non-progressive disease have a significantly greater reduction in mean Ki (day 2: p=0.004; day 28: p=0.006). A 50 - 60% reduction in Ki is associated with non-progressive disease. Exposure is approximately 114 hr·µM based on exposure-response modeling. Accounting for a PK variability, a dose with the lower limit (95% Cl) of exposure at 114 hr·µM should be the optimal dose, and thus a daily dose of 1250 mg is recommended as the biologically active dose. Supporting the selected biologically active dose is the dose-dependent acute rise in plasma VEGF level in patients with non-progressive disease. Responders who received doses of >1000 mg/day achieved up to 5 fold rise in plasma VEGF levels within the first 28 days of treatment.

## Claims

1. Use of a 4-pyridylmethyl-phthalazine agent for the manufacture of a pharmaceutical preparation for the treatment of renal carcinoma.

2. Use of a 4-pyridylmethyl-phthalazine agent for the manufacture of a pharmaceutical preparation for the inhibition of metastatic growth in a patient with a renal carcinoma.

3. The use of claim 1 or 2 wherein the 4-pyridylmethyl-phthalazine agent is 1-(4-chloroanilino)-4-(4-pyridylmethyl)phthalazine or a pharmaceutically acceptable salt thereof.

4. The use of claim 1 or 2 wherein the pharmaceutically effective amount is from 300 mg to 2000 mg administered daily.

5. The use of claim 1 or 2 wherein the renal carcinoma is metastatic renal carcinoma.

## Patentansprüche

1. Verwendung eines 4-Pyridylmethylphthalazinwirkstoffs zur Herstellung eines pharmazeutischen Präparats für die Behandlung von Nierenkarzinom.

2. Verwendung eines 4-Pyridylmethylphthalazinwirkstoffs zur Herstellung eines pharmazeutischen Präparats für die Inhibition eines Metastasenwachstums bei einem Patienten mit einem Nierenkarzinom.

3. Verwendung nach Anspruch 1 oder 2, worin der 4-Pyridylmethylphthalazinwirkstoff 1-(4-Chloranilino)-4-(4-pyridylmethyl)-phthalazin oder ein pharmazeutisch akzeptables Salz hiervon ist.

4. Verwendung nach Anspruch 1 oder 2, worin die pharmazeutisch wirksame Menge eine täglich verabreichte Menge von 300 mg bis 2000 mg ist.

5. Verwendung nach Anspruch 1 oder 2, worin das Nierenkarzinom ein metastatisches Nierenkarzinom ist.

## Revendications

1. Utilisation d'un agent 4-pyridylméthylphtalazine pour la fabrication d'une préparation pharmaceutique destinée au traitement du carcinome rénal.

2. Utilisation d'un agent 4-pyridylméthylphtalazine pour la fabrication d'une préparation pharmaceutique destinée à inhiber la croissance métastatique chez un patient atteint d'un carcinome rénal.

3. Utilisation selon la revendication 1 ou 2, dans laquelle l'agent 4-pyridylméthylphtalazine est de la 1-(4-chloroanilino)-4-(4-pyridylméthyl)phtalazine ou l'un de ses sels acceptable sur le plan pharmaceutique.

4. Utilisation selon la revendication 1 ou 2, dans laquelle la quantité efficace sur le plan pharmaceutique est de 300 mg à 2000 mg, administrés quotidiennement.

5. Utilisation selon la revendication 1 ou 2, dans laquelle le carcinome rénal est un carcinome rénal métastatique.
